(19) Europäisches Patentamt
European Patent Office
Office européen des brevets

(11) Publication number: 0 338 824
A2

# (12) EUROPEAN PATENT APPLICATION

(21) Application number: 89303920.6

(22) Date of filing: 20.04.89

(51) Int. Cl.[4]: C 07 D 499/32
C 07 D 499/70, A 61 K 31/43

(30) Priority: 22.04.88 GB 8809534

(43) Date of publication of application:
25.10.89 Bulletin 89/43

(84) Designated Contracting States:
BE CH DE FR GB IT LI NL

(71) Applicant: BEECHAM GROUP PLC
Beecham House Great West Road
Brentford Middlesex TW8 9BD (GB)

(72) Inventor: Best, Desmond John
Beecham Pharmaceuticals Brockham Park
Betchworth Surrey RH3 7AJ (GB)

Osborne, Neal Frederick
Beecham Pharmaceuticals Brockham Park
Betchworth Surrey RH3 7AJ (GB)

(74) Representative: West, Vivien et al
Beecham Pharmaceuticals Great Burgh Yew Tree
Bottom Road
Epsom Surrey KT18 5XQ (GB)

The title of the invention has been amended (Guidelines for Examination in the EPO, A-III, 7.3).

(54) **Esters of 6-beta-(alpha-oxyimino)-acylamino-penicillanic acid derivatives.**

(57) Compounds of formula (I):

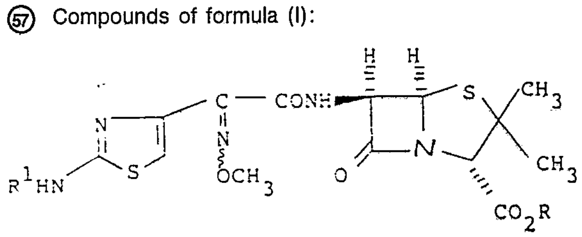

(I)

wherein $R^1$ is hydrogen or an amino protecting group and R is a group $CHR^a OCOR^b$, wherein $R^a$ is hydrogen, $C_{1-6}$ alkyl, $C_{3-7}$ cycloalkyl or phenyl, and $R^b$ is $C_{1-6}$ alkyl, $C_{1-6}$ alkoxy, phenyl optionally substituted with $-NHCOCHR^cNH_2$, benzyl, $C_{3-7}$ cycloalkyl, or $C_{1-6}$ alkyl $C_{3-7}$ cycloalkyl; or $R^a$ and $R^b$ together form a 1,2-phenylene group optionally substituted by one or two methoxy groups,wherein $R^c$ represents hydrogen or lower alkyl, are disclosed, having antibacterial properties.

EP 0 338 824 A2

**Description**

## ANTIBACTERIAL COMPOUNDS

This invention relates to novel β-lactam containing compounds, their preparation and their use, and in particular to a novel class of penicillins. These compounds have antibacterial properties, and therefore are of use in the treatment of bacterial infections in humans and animals caused by a wide range of organisms.

GB-A-1 399 087 discloses a novel class of penicillin antibiotics containing a 6β-(α-etherified oxyimino)-acylamino group.

EP-A-0 210 815 discloses a compound of formula (A) or a pharmaceutically acceptable salt or in-vivo hydrolysable ester thereof:

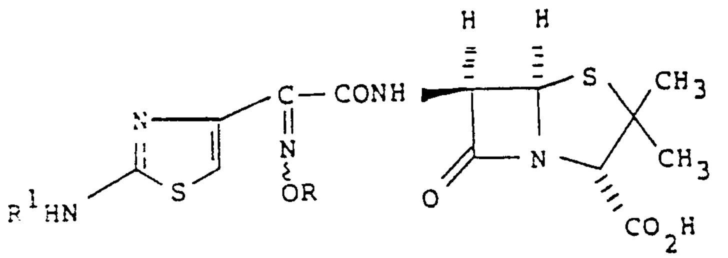

(A)

wherein $R^1$ is hydrogen or an amino protecting group and R is substituted methyl; optionally substituted $C_{2-12}$ alkyl, alkenyl or alkynyl; carbocyclyl; aryl or heterocyclyl.

DE-A-2 826 482 discloses a compound of formula (B):

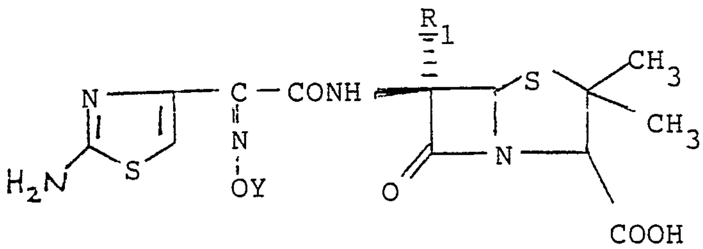

(B)

wherein $R_1$ is H or $CH_3O$ and Y is H or $CH_3$ .

The present invention provides a compound of formula (I):

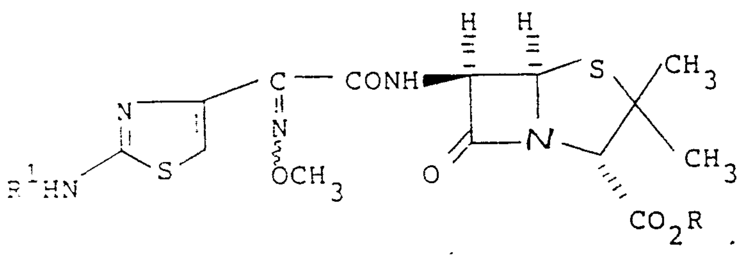

(I)

wherein $R^1$ is hydrogen or an amino protecting group and R is a group $CHR^a OCOR^b$, wherein $R^a$ is hydrogen, $C_{1-6}$ alkyl, $C_{3-7}$ cycloalkyl or phenyl, and Rb is $C_{1-6}$ alkyl, $C_{1-6}$ alkoxy, phenyl optionally substituted with $-NHCOCHR^cNH_2$, benzyl, $C_{3-7}$ cycloalkyl, or $C_{1-6}$ alkyl $C_{3-7}$ cycloalkyl; or $R^a$ and $R^b$ together form a 1,2-phenylene group optionally substituted by one or two methoxy groups,wherein $R^c$ represents hydrogen or lower alkyl.

Examples of suitable groups R include, for example, acyloxyalkyl groups such as acetoxymethyl, pivaloyloxymethyl, α-acetoxyethyl, α-pivaloyloxyethyl, and 1-(cyclohexylcarbonyloxy)prop-1-yl; alkoxycarbonyloxyalkyl groups, such as ethoxycarbonyloxymethyl and α-ethoxycarbonyl oxyethyl; and lactone groups such as phthalidyl and dimethoxyphthalidyl.

Compounds of the invention may exist in two or more tautomeric forms, e.g. those having the partial structures below:

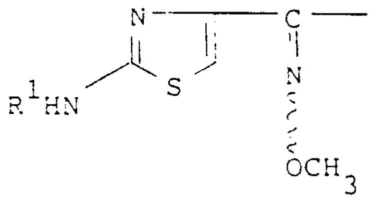

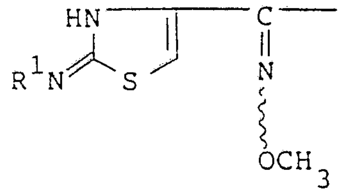

It should be understood that all tautomeric forms of the compound of formula (I) are included within the scope of the invention.

Suitable amino protecting groups $R^1$ are those well known in the art which may be removed under conventional conditions without disruption of the remainder of the molecule.

Examples of amino protecting groups $R^1$ include $C_{1-6}$ alkanoyl; benzoyl; benzyl optionally substituted in the phenyl ring by one or two substituents selected from $C_{1-4}$ alkyl, $C_{1-4}$ alkoxy, trifluoromethyl, halogen, or nitro; $C_{1-4}$ alkoxycarbonyl; benzyloxycarbonyl or trityl substituted as for benzyl above; allyloxycarbonyl, trichloroethoxycarbonyl or chloroacetyl.

The term 'halogen' refers to fluorine, chlorine, bromine and iodine.

Some of the compounds of this invention may be crystallised or recrystallised from conventional organic solvents such as lower alcohols, hydrocarbons and esters such as ethyl acetate In such cases solvates may be formed. This invention includes within its scope stoichiometric solvates including hydrates as well as compounds containing variable amounts of water that may be produced by processes such as lyophilisation.

Since the compounds of the formula (I) are intended for use in pharmaceutical compositions it will readily be understood that they are each provided in substantially pure form, for example at least 60% pure, more suitably at least 75% pure and preferably at least 85%, especially at least 95% pure (% are on a weight for weight basis). Impure preparations of the compounds of the formula (I) may be used for preparing the more pure forms used in the pharmaceutical compositions; these less pure preparations of the compounds of formula (I) should contain at least 1%, more suitably at least 5% and preferably from 10 to 49% of a compound of the formula (I).

Compounds of the present invention may exist as either syn or anti isomers, or may exist as mixtures of syn and anti isomers containing at least 75% of one such isomer, or preferably at least 90% of one such isomer.

Herein the terms syn and anti refer to the configuration of the group $OCH_3$ with respect to the carboxamido group, the syn-configuration (sometimes called the Z-configuration) being denoted thus:

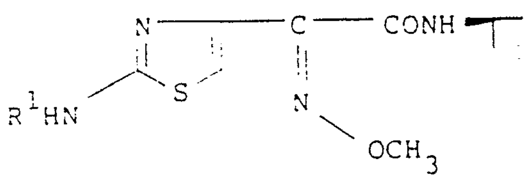

and the anti configuration (sometimes called the E-configuration) being denoted thus:

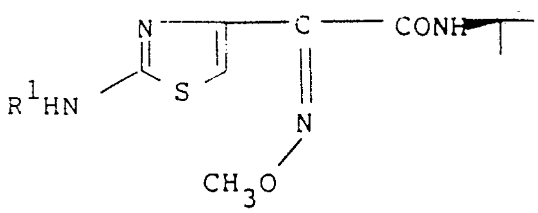

Preferred compounds of the present invention are the syn-isomers of the formula (II):

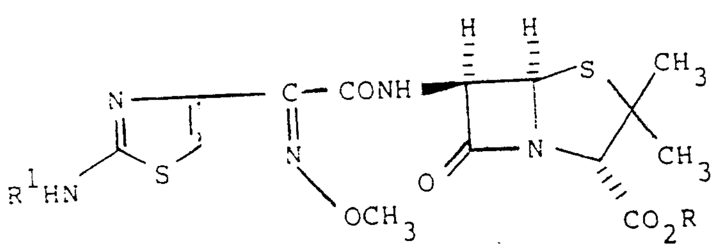

(II)

wherein R and $R^1$ are as hereinbefore defined.

The compounds of the formula (I) may be prepared by esterifying 6β-[2-(2-aminothiazol-4-yl)-(Z)-methoxyimino)acetamido]penicillanic acid or a salt thereof with a compound of the formula $XCHR^aOCOR^b$ in which $R^a$ and $R^b$ are as hereinbefore defined and X is a leaving group.

Examples of suitable leaving groups X include halogen as hereinbefore defined and particularly suitable examples are iodine and bromine.

Suitable salts of the penicillanic acid include sodium, potassium, silver, and amine salts eg. triethylamine, diisopropylethylamine and the like.

The reaction may be carried out at non extreme temperature in conventional solvents such as dimethylformamide, dimethyl sulphoxide, acetonitrile, and the like. In the case of sodium and potassium salts of the penicillanic acid it may be of advantage to carry out the reaction in the presence of a cationic chelation agent such as a crown ether eg 15-crown-5, 18 crown-6. Alternatively the reaction may be carried out using phase transfer catalysis methods in aqueous and/or organic solutions in the presence of a quaternary ammonium salt such as tetrabutyl ammonium hydrogensulphate and the like. Suitable organic solvents include dichloromethane and the like.

In an alternative process the compound of formula (I) in which $R^a$ is hydrogen may be prepared by reacting a compound of the formula (Ia) with an acid $R^bCO_2H$ or salt thereof:

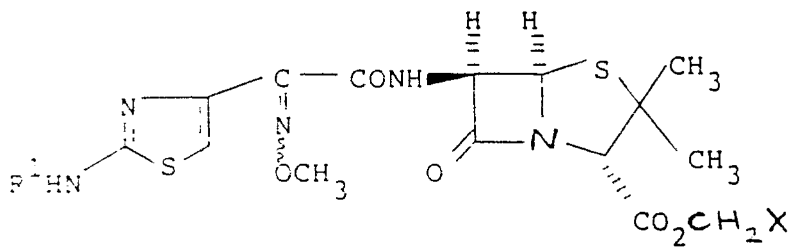

(Ia)

in which X, $R^1$, and $R^b$ are as hereinbefore defined. Suitable salts, solvents and conditions for carrying out the reaction are as hereinbefore defined.

Compounds of the formula (Ia) may be prepared by reacting 6β-[2-(2-aminothiazol-4-yl)-(Z)-methoxyimino)acetamido] penicillanic acid or a salt thereof with a compound of the formula $X^1CH_2Cl$ in which $X^1$ is iodine or a group $-OSO_2Cl$.

Suitable salts, solvents and conditions for carrying out the reaction are as hereinbefore defined.

It may be advantageous to convert a compound of formula (Ia) in which X is chlorine to a compound of formula (Ia) in which X is a different leaving group, e.g. iodine, prior to reaction with a compound of formula $R^bCO_2H$. Such transformations may be conveniently carried out by the reaction of a compound of the formula (Ia) in which X is chlorine with anhydrous metal iodide, eg sodium or potassium, in an anhydrous organic solvent. Suitable organic solvents include acetone, acetonitrile and the like.

The compounds of the formula (Ia) are novel intermediates and constitute a further subject of the present invention.

The free acid starting material may be prepared using processes analagous to those described in EP-A-0 210 815.

The present invention also provides a pharmaceutical composition which comprises a compound of formula (I) and a pharmaceutically acceptable carrier. The compositions of the invention include those in a form adapted for oral, topical or parenteral use and may be used for the treatment of the infection in mammals including humans.

The antibiotic compounds according to the invention may be formulated for administration in any convenient way for use in human or veterinary medicine, by analogy with other antibiotics, and the invention therefore includes within its scope a pharmaceutical composition comprising a compound of formula (I) above together with a pharmaceutical carrier or excipient.

The composition may be formulated for administration by any route, such as oral, topical or parenteral. The compositions may be in the form of tablets, capsules, powders, granules, lozenges, creams or liquid preparations, such as oral or sterile parenteral solutions or suspensions.

The topical formulations of the present invention may be presented as, for instance, ointments, creams or lotions, eye ointments and eye or ear drops, impregnated dressings and aerosols, and may contain appropriate conventional additives such as preservatives, solvents to assist drug penetration and emollients in ointments and creams.

The formulations may also contain compatible conventional carriers, such as cream or ointment bases and ethanol or oleyl alcohol for lotions. Such carriers may be present as from about 1% up to about 98% of the formulation. More usually they will form up to about 80% of the formulation.

Tablets and capsules for oral administration may be in unit dose presentation form, and may contain conventional excipients such as binding agents, for example syrup, acacia, gelatin, sorbitol, tragacanth, or polyvinylpyrollidone: fillers, for example lactose, sugar, maize-starch, calcium phosphate, sorbitol or glycine, tabletting lubricants, for example magnesium stearate, talc, polyethylene glycol or silica; disintegrants, for example potato starch; or acceptable wetting agents such as sodium lauryl sulphate. The tablets may be

coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and, if desired, conventional flavouring or colouring agents.

Suppositories will contain conventional suppository bases, e.g. cocoa-butter or other glyceride.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing. Advantageously, agents such as a local anaesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection may be supplied to reconstitute the liquid prior to use. Parental suspensions are prepared in substantially the same manner except that the compound is suspended in the vehicle instead of being dissolved and sterilization cannot be accomplished by filtration. The compound can be sterilised by exposure to ethylene oxide before suspending in the sterile vehicle. Advantageously, a surfactant or wetting agent is included in the composition to facilitate uniform distribution of the compound.

The compositions may contain from 0.1% by weight, preferably from 10-60% by weight, of the active material, depending on the method of administration. Where the composition comprise dosage units, each unit will preferably contain from 50-500 mg of the active ingredient. The dosage as employed for adult human treatment will preferably range from 100 to 3000 mg per day, for instance 1500 mg per day depending on the route and frequency of administration. Such a dosage corresponds to 1.5 to 50 mg/kg per day. Suitably the dosage is from 5 to 20 mg/kg per day.

No toxicological effects are indicated when a compound of formula (I) is administered in the above-mentioned dosage range.

The compound of the invention of formula (I) may be used as the sole therapeutic agent in compositions of the invention or may be used in combination with other antibiotics or with a β-lactamase inhibitor.

Advantageously the compositions also comprise a compound of formula (III) or a pharmaceutically acceptable salt or ester thereof:

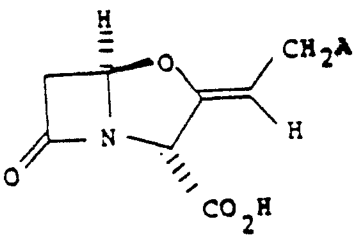

(III)

wherein A is hydroxyl; substituted hydroxyl; thiol; a group of formula $SO_2R^2$ wherein $R^2$ is $C_{1-6}$ alkyl; substituted thiol; amino; mono- or di-hydrocarbyl substituted amino; mono- or di-acylamino; an optionally substituted triazolyl group; or an optionally substituted tetrazolyl group as described in EP O 053 893.

A further advantageous composition comprises an antibiotic compound according to the invention and a pharmaceutically acceptable carrier or excipient together with a β-lactamase inhibitor of formula (IV) or a pharmaceutically acceptable salt or <u>in vivo</u> hydrolysable ester thereof:

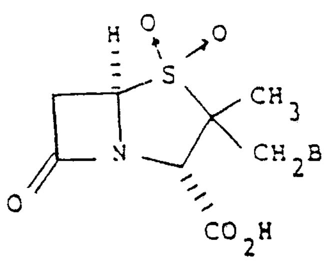

(IV)

wherein B is hydrogen, halogen or a group of formula:

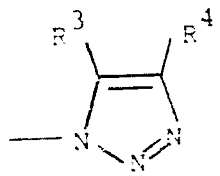

in which $R^3$ and $R^4$ are the same or different and each is hydrogen, $C_{1-6}$ alkoxycarbonyl, or carboxy or a pharmaceutically acceptable salt thereof.

Further suitable β-lactamase inhibitors include 6-alkylidene penem of formula V below:

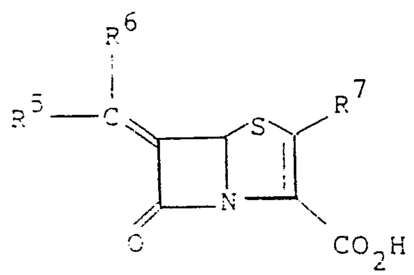

(V)

or a pharmaceutically acceptable salt or in vivo hydrolysable ester thereof, wherein $R^5$ and $R^6$ are the same or different and each represents hydrogen, or a $C_{1-10}$ hydrocarbon or heterocyclic group optionally substituted with a functional group; and $R^7$ represents hydrogen or a group of formula $R^d$ or $-SR^d$ where $R^d$ is an optionally substituted $C_{1-10}$ hydrocarbon or heterocyclic group, as described in European Patent Application No, 81301683.9 (Publication Number 0 041 768).

Other suitable β-lactamase inhibitors include 6β-bromopenicillanic acid and salts and in vivo hydrolysable esters thereof and 6β-iodopenicillanic acid and salts and in vivo hydrolysable esters thereof.

Such compositions of this invention comprising a β-lactamase inhibitor are formulated in conventional manner.

The present invention also includes a method of treating bacterial infections in humans and animals which comprises the administration of a therapeutically effective amount of an antibiotic compound of this invention.

Antibiotic compounds of the present invention are active against a broad range of bacteria, in particular they are useful for treatment of respiratory tract and urinary tract infections in humans and mastitis in cattle.

The following Examples illustrate the preparation of the compounds of the present invention.

## EXAMPLE 1.

6β-[2-(2-Aminothiazol-4-yl)-(Z)-2-(methoxyimino)acetamido]Penicillanic acid.

Methanesulphonyl chloride (2.71ml) was added to a mixture of 2-(2-aminothiazol-4-yl)-(Z)-2-(methoxyimino) acetic acid (6.4g) and diisopropylethylamine (6.09ml) in dry dimethylformamide (40ml) at -60°C. After stirring at -50 to -60°C for 40 minutes the mixture was added to a stirred mixture of 6-aminopenicillanic acid (6.26g) and triethylamine (11.1ml) in water (40ml) at 0°C. After stirring at 0°C for 15 minutes the pH of the mixture was adjusted to 7.0. The mixture was evaporated and the residue re-evaporated several times from xylene. The residue was dissolved in water, filtered, and chromatographed on Diaion HP20SS eluting with tetrahydrofuran/ water mixtures (gradient elution 0.5 to 5.0%). The appropriate fractions were freeze dried to give the title compound as an approximately 2:1 mixture of diisopropylethylamine and triethylamine salts (5.49g), $\delta_H$ ($D_2O$) 1.49 (3H, s), 1.58 (3H, s), 3.94 (3H, s), 4.22 (1H, s), 5.58-5.61 (2H, m), 7.00 (1H, s). Additional signals at 1.20-1.32 (m), 3.15 (q, J 7.4Hz), and 3.67 (h, J6.6Hz) integrated for a 2:1 ratio of diisopropylethylammonium and triethylammonium ions.

## EXAMPLE 2

[R,S]-Phthalid-3-yl 6,β-[2-(2-aminothiazol-4-yl)-(Z)-2-(methoxyimino)acetamido]penicillanate.

A mixture of [R,S]-3-bromophthalide (213mg) and 6,β,[2-(2-aminothiazol-4-yl)-(Z)-2-(methoxyimino)acetamido]penicillanic acid (a 2:1 mixture of diisopropylethyl amine and triethylamine salts, 528mg) in dry N,N-dimethylformamide was stirred at room temperature for 2h. The solution was diluted with ethyl acetate, washed with water, dried ($MgSO_4$) and the solvent evaporated under reduced pressure. The title comoound (330mg) was isolated by flash chromatography on silica eluting with ethyl acetate : cyclohexane, $\nu_{max}$ (KBr) 3337, 2973, 1786, 1676, 1612, 1529 and 979cm$^{-1}$, &H [(CD3)2CO] 1.62, 1.67 (3H, 2s), 3.92 (3H, s), 4.56, 4.58 (1H, 2s), 5.13-5.87 (2H, m), 6.77 (2H, s), 6.86 (1H, s), 7.65 (1H, s) 7.77-8.03 (4H, m), 8.33 (1H, d).

EXAMPLE 3.

[R,S]-1-Acetoxyethyl 6,B-[2-(2-aminothiazol-4-yl)-(Z)2-(methoxyimino)-acetamidolpenicillanate

6,β-[2-(2-Aminothiazol-4-yl)-(Z)-2-(methoxyimino)acetamido]penicillanic acid (a 2:1 mixture of diisopropylethylamine and triethylamine salt, 290mg) was esterified with [R,S]-1-acetoxy-1-bromoethane (84mg),as described in Example 2 to give the title compound (85mg), $\nu_{max}$ (KBr) 3332, 2936, 1765, 1676, 1617, 1533 and 1077cm$^{-1}$, $\delta_H$ [$(CD_3)_2CO$] 1.52, 1.53, 1.55, 1.63, 1.65 (9H, 5s), 2.07 (3H, s), 3.90 (3H, s), 4.38, 4.42 (1H, 2s), 5.62-5.87 (2H, m), 6.68 (2H, s), 6.88 (1H, m), 8.24 (1H, d). [Mass spectrum : +ve ion (thioglycerol) $MH^+$ (486)].

EXAMPLE 4

a) 2-[N-(4-Nitrobenzyloxycarbonyl)glycylamino] benzoic acid.

Oxalyl chloride (1.75ml) was added, dropwise over 2 minutes to a stirred solution of dry dimethylformamide (1.75ml) in dry dichloromethane (30ml) at -20°C under dry nitrogen. The stirred mixture was allowed to attain 0°C during 15 minutes, recooled to -20°C, and treated, portionwise over 1 minutes, with N-(4-nitrobenzyloxycarbonyl)glycine (5.08g). The stirred mixture was allowed to attain 0°C during 30 minutes, re-cooled to -20°C, and treated, dropwise over 10 minutes, with a solution containing 2-aminobenzoic acid (3.74g) and triethylamine (2.78ml) in dry dichloromethane (20ml). The cooling bath was removed and the mixture was stirred for 2h [after a few minutes a semi-solid gel formed which was dispersed by the addition of dichloromethane (50ml)]. The mixture was diluted with ethyl acetate (250ml) and was washed with 5% citric acid (25ml) and brine (3 x 25ml). The dried ($MgSO_4$) organic layer was evaporated and the residue was triturated with ether to give the title acid as an off white solid (6.64g), m.p. 188-190°C (microcrystalline solid ex acetone/hexane); $\nu_{max}$ (Nujol) 3300, 3500-2000 br, 1735, 1705, and 1680cm$^{-1}$; $\delta_H$ [$(CD_3)_2CO/(CD_3)_2SO$] 3.98 (2H, d, J 6Hz, collapses to s on exch. $D_2O$), 5.35 (2H, s), 6.8-8.6 (9H, m, 2H exch. $D_2O$), 8.85 (1H, d, J 8Hz), and 11.95 (1H, brs, exch. $D_2O$).

b) 2-[N-(4-Nitrobenzyloxycarbonyl)glycylamino]benzoyloxymethyl 6β-[2-(2-aminothiazol-4-yl)-(Z)-2-(methoxyimino)acetamido]penicillanate.

A solution of chloromethyl chlorosulphate (275mg) in dichloromethane (1ml) was added, dropwise over $\frac{1}{2}$ minute, to a stirred mixture of 2-[N-(4-nitrobenzyloxycarbonyl)glycylamino]benzoic acid (500mg), sodium bicarbonate (450mg), tetrabutylammonium hydrogensulphate (70mg), dichloromethane (10ml), and water (10ml). After stirring at room temperature for 1h the solid which had precipitated was redissolved by addition of dichloromethane (50ml). The organic layer was separated and was washed with dilute brine (2 x 10ml), dried ($MgSO_4$), and evaporated to give the crude product, chloromethyl 2-[N-(4-nitrobenzyloxycarbonyl)glycylamino]benzoate, as a solid.

Without further purification, the crude chloromethyl ester was dissolved in dry acetone (50ml) and was treated with sodium iodide (2.08g). After stirring at room temperature for 18h the mixture was evaporated. The reside was dissolved in ethyl acetate (50ml) and was washed with water (3 x 10ml), dried ($MgSO_4$), and evaported to give the crude product, iodomethyl 2-[N-(4-nitrobenzyloxycarbonyl)glycylamino]benzoate, as a solid.

Without further purification the crude iodomethyl ester was dissolved in dry dimethylformamide (10ml) and treated with 6β-[2-(2-aminothiazol-4-yl)-(Z)-2-methoxyimino)acetamido]penicillanic acid (a 2:1 mixture of diisopropylethylamine and triethylamine salts, 730mg). After stirring at room temperature for 4h the mixture was diluted with ethyl acette (50ml) and was washed with 5% citric acid (10ml), brine (10ml), saturated $NaHCO_3$ (10ml), and brine (3 x 10ml). The dried ($MgSO_4$) organic layer was evaporated and the residue chromatographed on silica gel eluting with ethyl acetate/hexane mixtures to give the title compound as an amorphous solid (502mg), $\nu_{max}$ ($CHCl_3$) 3600-3100, 1785, 1730, 1700, and 1675cm$^{-1}$; $\delta_H$ [$(CD_3)_2CO$] 1.47 (3H, s), 1.61 (3H, s), 3.88 (3H, s), 4.03 (2H, d, J 6.2Hz, collapses to s on exch. $D_2O$), 4.49 (1H, s), 5.37 (2H, s), 5.67 (1H, d, J 4.1Hz), 5.76 (1H, dd, J 8.0 and 4.1Hz, collapses to d, J 4.1Hz, on exch. $D_2O$), 6.12 and 6.16 (2H, ABq, J 6.0Hz), 6.64 (2H, s, exch. $D_2O$), 6.85 (1H, s), 7.17-7.45 (2H, m, 1H exch. $D_2O$), 7.62-7.74 (m) and 7.77 (d, J 7.4Hz), together 3H, 8.07 (1H, d, J 7.8Hz), 8.18-8.37 (3H, m, 1H exch. $D_2O$), 8.76 (1H, d, J 8.5Hz), 11.28 (1H, s, exch. $D_2O$).

c) 2-Glycylaminobenzoyloxymethyl 6β-[2-(2-amino thiazol-4-yl)-(Z)-2-(methoxyimino)acetamido]penicillanate hydrochloride.

A solution of 2-[N-(4-nitrobenzyloxycarbonyl)glycylamino]benzyloxymethyl 6β-[2-(2-aminothiazol-4-yl)-(Z)-2-(methoxyimino)acetamido]penicillanate (250mg) in a mixture of 1,4-dioxane (20ml) and water (20ml) was hydrogenated over 5% palladium/charcoal (375mg) at S.T.P. for 30 minutes. The mixture was filtered through Kieselguhr and the residue was washed with 50% aqueous dioxane (2 x 10ml). The combined filtrates were treated with hydrochloric acid (3.19ml of 0.1M) and were kept at room temperature for 6h. The mixture was diluted with ethyl acetate (25ml) and filtered through Kieselguhr. The aqueous layer was separated and the organic layer was re-extracted with water (5ml). The combined aqueous layers were washed with ethyl acetate (10ml) and ether (2 x 10ml), concentrated, and freeze-dried to give the title compound as a pale yellow solid (98mg), $\nu_{max}$ (KBr) 3700-2150, 1775, 1699, 1675sh, 1630, 1609, 1591cm$^{-1}$; $\delta_H$ ($D_2O$) 1.47 (3H, s), 1.64 (3H,

s), 4.05 (3H, s), 4.14 (2H, s), 5.67 (1H, d, J 4.1Hz), 5.77 (1H, d, J 4.1Hz), 6.12 and 6.17 (2H, ABq, J 6.1Hz), 7.13 (1H, s), 7.39-7.45 (1H, m), 7.72-7.79 (1H, m), 7.93 (1H, d, J 8.0Hz), 8.09 (1H, dd, J 8.0 and 1.5Hz).

## Claims

1. A compound of formula (I):

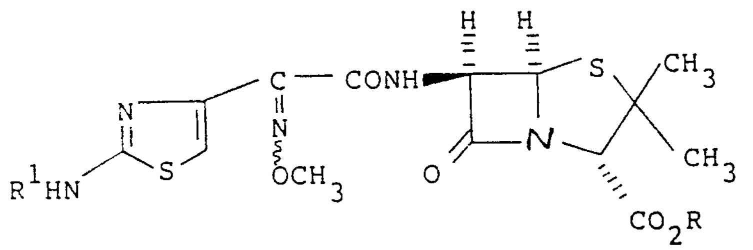

(I)

wherein $R^1$ is hydrogen or an amino protecting group and R is a group $CHR^a$ $OCOR^b$, wherein $R^a$ is hydrogen, $C_{1-6}$ alkyl, $C_{3-7}$ cycloalkyl or phenyl, and $R^b$ is $C_{1-6}$ alkyl, $C_{1-6}$ alkoxy, phenyl optionally substituted with $-NHCOCHR^cNH_2$, benzyl, $C_{3-7}$ cycloalkyl, or $C_{1-6}$ alkyl $C_{3-7}$ cycloalkyl; or $R^a$ and $R^b$ together form a 1,2-phenylene group optionally substituted by one or two methoxy groups,wherein $R^c$ represents hydrogen or lower alkyl.

2. A compound according to claim 1 in which the group R is selected from acetoxymethyl, pivaloyloxymethyl, α-acetoxyethyl, α-pivaloyloxyethyl, 1-(cyclohexylcarbonyloxy)prop-1-yl; ethoxycarbonyloxymethyl, α-ethoxycarbonyl oxyethyl, phthalidyl and dimethoxyphthalidyl.

3. A compound according to claim 1 in which the amino protecting group $R^1$ is selected from $C_{1-6}$ alkanoyl; benzoyl; benzyl optionally substituted in the phenyl ring by one or two substituents selected from $C_{1-4}$ alkyl, $C_{1-4}$ alkoxy, trifluoromethyl, halogen, or nitro; $C_{1-4}$ alkoxycarbonyl; benzyloxycarbonyl or trityl substituted as for benzyl above; allyloxycarbonyl, trichloroethoxycarbonyl and chloroacetyl.

4. A compound according to claim 1 selected from [R,S]-Phthalid-3-yl 6,β-[2-(2-aminothiazol-4-yl)-(Z)-2-(methoxyimino)acetamido]penicillanate;
[R,S]-1-Acetoxyethyl 6,β-[2-(2-aminothiazol-4-yl)-(Z)-2-(methoxyimino)-acetamido]penicillanate; and
2-Glycylaminobenzoyloxymethyl 6β-[2-(2-aminothiazol-4-yl)-(Z)-2-(methoxyimino)acetamido]penicillanate hydrochloride.

5. A pharmaceutical composition comprising an antibiotically effective and non-toxic amount of a compound according to claim 1 and a pharmaceutically effective carrier.

6. A pharmaceutical composition according to claim 5, further comprising a β-lactamase inhibitor.

7. A method of preparing a compound of the formula (I) as claimed in any one of claims 1 to 4 which comprises esterifying 6β-[2-(2-aminothiazol-4-yl)-(Z)-methoxyimino)acetamido]penicillanic acid or a salt thereof with a compound of the formula $XCHR^aOCOR^b$ in which $R^a$ and $R^b$ are as hereinbefore defined and X is a leaving group.

8. A method of preparing a compound of formula (I) in which $R_a$ is hydrogen as claimed in any one of claims 1 to 4 which comprises reacting a compound of the formula (Ia) with an acid $R^bCO_2H$ or salt thereof:

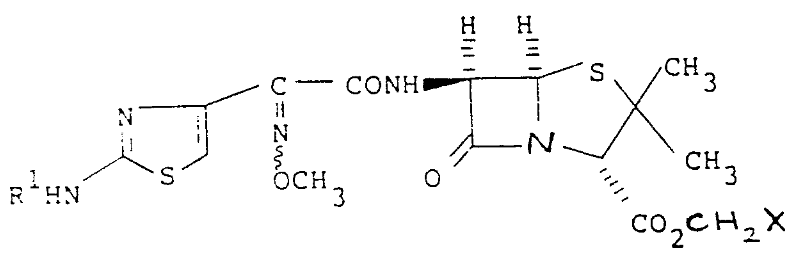

(Ia)

where X is a leaving group and $R^1$ and Rb are as hereinbefore defined.

9. A compound of formula (Ia) as defined in claim 8.

10. The use of a compound as claimed in any one of claims 1 to 4 for the manufacture of a medicament for the treatment of bacterial infections in humans and animals.